Europäisches Patentamt

European Patent Office

Office européen des brevets

(19)

(11) Numéro de publication : **0 011 897**
**B1**

(12) FASCICULE DE BREVET EUROPÉEN

(45) Date de publication du fascicule du brevet :
30.07.86

(21) Numéro de dépôt : **79200683.5**

(22) Date de dépôt : **21.11.79**

(51) Int. Cl.⁴ : **A 61 B   6/02**

(54) **Appareil d'examen tomographique par exploration de milieux aux rayons X ou gamma.**

(30) Priorité : **27.11.78 FR 7833430**

(43) Date de publication de la demande :
**11.06.80 Bulletin 80/12**

(45) Mention de la délivrance du brevet :
**30.07.86 Bulletin 86/31**

(84) Etats contractants désignés :
**DE FR GB IT NL SE**

(56) Documents cités :
**DE-A- 2 655 230**
**FR-A- 2 386 055**
**US-A- 4 053 779**

(73) Titulaire : **Laboratoires d'Electronique et de Physique Appliquée L.E.P.**
**3, Avenue Descartes**
**F-94450 Limeil-Brévannes (FR)**
**FR**
**N.V. Philips' Gloeilampenfabrieken**
**Groenewoudseweg 1**
**NL-5621 BA Eindhoven (NL)**
**DE GB IT NL SE**

(72) Inventeur : **Jatteau, Michel**
**Société Civile S.P.I.D. 209, Rue de l'Université**
**F-75007 Paris (FR)**
Inventeur : **Chalmeton, Vincent**
**Société Civile S.P.I.D. 209, Rue de l'Université**
**F-75007 Paris (FR)**
Inventeur : **Pauvert, Joseph**
**Société Civile S.P.I.D. 209, Rue de l'Université**
**F-75007 Paris (FR)**

(74) Mandataire : **Landousy, Christian et al**
**Société Civile S.P.I.D. 209, Rue de l'Université**
**F-75007 Paris (FR)**

Jouve, 18. rue St-Denis, 75001 Paris, France

# 0 011 897

## Description

La présente invention concerne un appareil d'examen tomographique aux rayons X ou gamma du type défini dans le préambule de la revendication 1 pour le contrôle de la structure interne d'un objet tridimensionnel. Plus particulièrement, l'appareil conforme à l'invention est destiné à permettre la représentation d'images de tranches de cet objet à l'aide de cartes de la densité d'électrons dans des éléments de volume de ces tranches, c'est-à-dire par une méthode densitométrique.

La radiologie conventionnelle donne d'un objet à trois dimensions une image plane résultant de la superposition, par projection sur un support, des « ombres portées » des diverses structures internes à cet objet et traversées par un faisceau incident de rayons X. Le faisceau qui émerge du corps du patient, s'il s'agit d'un examen d'organes, contient toute une série d'informations traduisant l'absorption du faisceau incident, plus ou moins importante selon les différences de forme, d'épaisseur et de nature de tous les organes rencontrés et selon la longueur d'onde du rayonnement. Sur le film ou l'écran destinés à recevoir le faisceau émergent porteur de ces informations, les différences de forme apparaissent en général avec une netteté satisfaisante et un contraste assez prononcé, mais il n'en est pas de même pour les différences d'épaisseur ou de nature. Pour pallier ces insuffisances, les radiologistes procèdent à certaines interventions, telles que des opacifications ou des injections de substances, mais dont la plupart sont pénibles pour le patient.

Le but de l'invention est de proposer un appareil d'examen tomographique aux rayons X ou gamma qui, sans recourir à des interventions désagréables pour un patient ni à une irradiation excessive et donc nocive pour celui-ci, permette de réaliser rapidement l'examen souhaité, la brièveté des temps de calcul des densités d'électrons étant obtenue tout en effectuant ces calculs de façon rigoureuse.

L'invention consiste à cet effet dans un appareil d'examen tomographique du type défini dans la revendication 1.

L'utilisation de cet appareil de tomographie densitométrique par utilisation de la diffusion de photons s'avère avantageuse à plusieurs égards. En effet, contrairement au cas de la tomographie par transmission d'un faisceau de rayons X ou gamma, dans lequel seul un très grand nombre de mesures d'atténuation permet à l'ordinateur, après des calculs complexes et assez longs, de reconstruire une carte des variations spatiales de la densité d'électrons dans les tissus examinés, le signal utile est ici constitué par un nombre de photons détectés (cette détection intervenant après diffusion à l'intérieur d'un élément de volume) directement proportionnel aux variations de densité que l'on cherche à observer. Par ailleurs, la probabilité de détecter des photons diffusés ayant subi dans les tissus examinés de multiples diffusions dans la même direction de détection est faible, ce qui assure une relative insensibilité des mesures à ces phénomènes perturbateurs.

On notera également que le détecteur principal est stationnaire, ce qui supprime tous les problèmes mécaniques rencontrés généralement dans les dispositifs mobiles et les problèmes électroniques qui peuvent en résulter (stabilité des nombreux détecteurs portés par ces dispositifs mobiles), et qu'il permet la localisation de chaque photon détecté après diffusion, ce qui supprime la nécessité d'effectuer cette localisation à la suite d'un traitement complexe de reconstruction d'image. D'autre part, la simplicité des calculs et leur réalisation en temps réel, c'est-à-dire, pour une tranche déterminée de région examinée, pendant le temps d'acquisition des résultats qui vont servir pour les calculs concernant la tranche suivante, permettent de n'avoir recours qu'à une capacité utile de mémoire relativement faible. Les moyens informatiques utilisés s'avèrent donc simples, et par suite peu coûteux.

Le détecteur principal stationnaire, à collimateurs parallèles définissant autant d'axes de détection distincts pour le repérage de la position des photons détectés après diffusion, n'est pas nouveau en soi, mais sa combinaison avec les autres moyens de l'invention permet la mise en œuvre particulière de l'appareil selon l'invention. Un tel détecteur a été décrit avec ses caractéristiques essentielles pour la première fois dans le brevet des Etats-Unis d'Amérique US-A-3011057, et est bien connu de l'homme du métier, dans cette structure d'origine ou avec les perfectionnements qui l'ont depuis amélioré sans en modifier le principe.

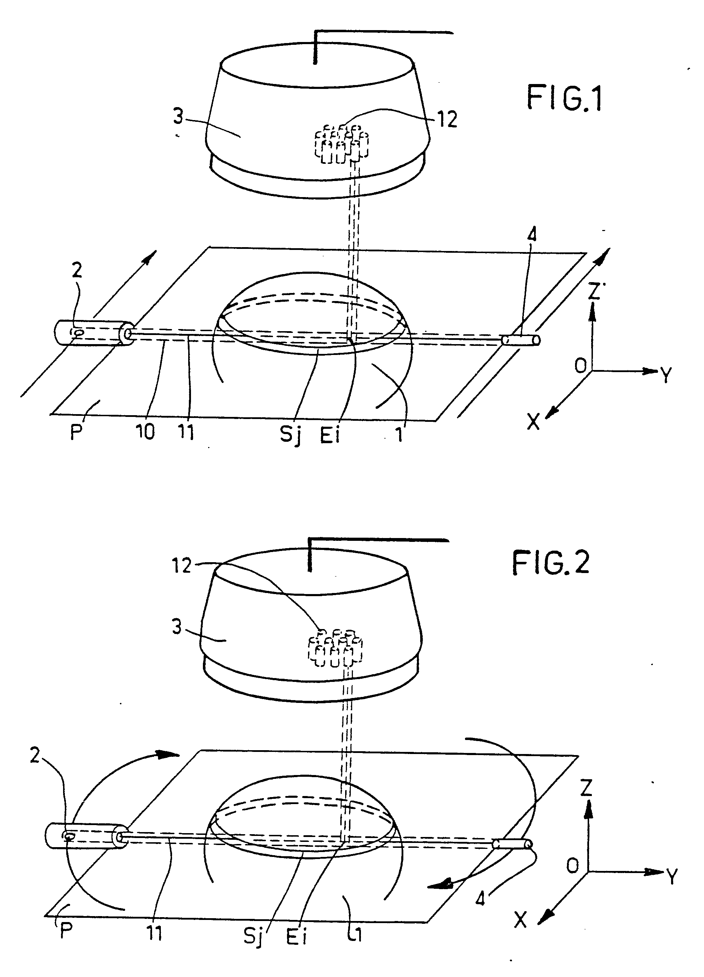

Par ailleurs, la demande de brevet français publiée sous la référence FR-A-2386055 décrit également un dispositif d'examen tomographique aux rayons X ou gamma du type défini dans le préambule de la revendication 1, mais des différences essentielles existent entre ce dispositif et l'appareil selon l'invention. En effet, dans le dispositif que décrit ce document, le dispositif de détection est constitué de plusieurs détecteurs élémentaires, mais il est absolument certain, d'après les informations apportées conjointement par la description et les figures 1 et 2, que ces détecteurs élémentaires concourent à la réalisation d'une seule mesure (et non d'autant de mesures que de détecteurs élémentaires). En outre, comme le précise ce document en page 6, lignes 25 à 28, cette mesure, se refusant expressément à privilégier un angle déterminé, car n'y voyant aucun avantage, est effectuée selon un angle quelconque ce qui implique que le dispositif de détection reçoit des photons diffusés d'énergies différentes selon l'angle de détection. D'autre part, il s'agit d'une mesure unique et simultanée de l'ensemble des photons diffusés selon un angle déterminé et selon son angle complémentaire à 180°, c'est-à-dire de photons d'énergies là encore différentes (un photon diffusé à 80°, par exemple, n'est pas porteur de la même énergie qu'un photon diffusé à 100°). Enfin, la vue en perspective de la figure 1 montre bien que, pour

2

cette mesure, on recueille, pour un angle déterminé, par exemple environ 80° qui est l'angle semble-t-il choisi sur la figure 2, l'ensemble des photons diffusés à partir du faisceau primaire dans un secteur angulaire défini par l'ouverture en forme de fente 7,7'.

Au contraire, dans le cas de la présente demande, l'appareil d'examen comprend, entre autres éléments, le détecteur stationnaire, c'est-à-dire un organe qui, comme le montre le brevet cité plus haut, se comporte comme un détecteur multiple de rayons, ou multidétecteur, qui fournit quasi-simultanément, pour tous les détecteurs élémentaires situés sur une ligne parallèle au faisceau émis par la source, autant de mesures du nombre de photons diffusés à 90° exclusivement par les éléments de volume correspondants (la partie de région traversée par le faisceau étant en effet ainsi divisée en autant d'éléments de volume ayant leurs dimensions définies à partir de celles des collimateurs), c'est-à-dire des photons de même énergie. En outre, on a bien précisé que l'on effectue une première mesure de ces photons diffusés dans une position déterminée de l'ensemble source-détecteur auxiliaire, puis une deuxième mesure distincte des photons diffusés, après permutation circulaire de la source et du détecteur ; en effet, ces deux résultats de mesure sont utilisés de façon bien distincte et séparée dans les calculs.

En définitive, on peut donc affirmer que le document cité et la présente demande reposent sur des principes de mesure différents. En effet, dans le premier cas, on veut recueillir à l'aide d'un diaphragme de forme appropriée (en l'occurrence, à ouverture en forme de fente couvrant un secteur angulaire le plus large possible et donc un nombre élevé de directions), le maximum de photons diffusés par un seul élément de volume de la région examinée, suivant un angle quelconque transversal au faisceau primaire (donc sans sélection énergétique puisque l'énergie des photons diffusés est différente pour chaque angle), et ce pour l'obtention d'un seul résultat de mesure par élément de volume (n'opérant pas la distinction entre les photons diffusés selon un certain angle et ceux diffusés selon l'angle complémentaire à 180°), en vue d'un effet technique premier qui est seulement d'obtenir à l'aide d'un balayage point par point l'image d'une tranche d'une région d'un corps par détermination de l'image de la répartition des densités d'électrons dans les régions explorés par le faisceau de rayonnement primaire (voir le document cité, page 2, lignes 24-27), c'est-à-dire une image des variations relatives de ces densités dans ladite région. Au contraire, dans la présente demande, on opère effectivement une quadruple sélection des photons diffusés, en n'acceptant que ceux qui sont diffusés (a) à 90° du faisceau émis par la source (b) dans une seule direction, celle qui est parallèle aux axes des collimateurs du détecteur stationnaire, (c) en éliminant ceux des photons qui sont diffusés plusieurs fois avant d'atteindre le détecteur stationnaire (d) et en distinguant la mesure du nombre des photons diffusés selon un certain angle et ceux diffusés selon l'angle complémentaire à 180°, et en vue d'un effet technique premier qui est l'établissement de « cartes » des valeurs exactes de la densité d'électrons des tissus dans la région examinée.

D'autres particularités et avantages de l'appareil conforme à l'invention seront mieux compris en se référant à la description qui va suivre, ainsi qu'aux dessins annexés qui montrent, à titre d'exemples non limitatifs, plusieurs modes de mise en œuvre de l'invention et dans lesquels :

les figures 1 et 2 sont des schémas aidant à comprendre le principe du procédé d'examen tomographique utilisé dans l'appareil selon l'invention, dans le cas d'un balayage rectiligne (figure 1) et dans celui d'un balayage circulaire (figure 2) autour de chaque tranche de région à examiner ;

la figure 3 correspond à la première phase de l'examen, c'est-à-dire à l'irradiation de la tranche de région à examiner la plus proche du détecteur principal ;

les figures 4 à 7 montrent les quatre types de mesures pouvant être effectuées par la mise en œuvre de l'appareil selon l'invention ;

la figure 8 montre de façon détaillée un premier mode de réalisation de l'appareil selon l'invention ;

les figures 9 à 11 montrent trois autres modes de réalisation de l'appareil selon l'invention ; et

la figure 12 montre une variante de réalisation du détecteur principal de rayonnement.

Le procédé d'examen tomographique décrit en se référant aux figures 1 à 7 et mis en œuvre dans l'appareil conforme à l'invention est destiné à permettre la représentation d'images de tranches d'un objet tridimensionnel 1. Pour cette mise en œuvre, on utilise essentiellement une source 2 de rayons X ou gamma, un détecteur principal stationnaire 3 et un détecteur auxiliaire 4, ainsi qu'un dispositif de balayage et de commande de balayage, un dispositif de traitement des résultats de détection, un dispositif de représentation des valeurs de densité d'électrons et un dispositif de commande générale de fonctionnement, qui seront décrits de façon plus détaillée dans la suite de la description.

La source 2, équipée d'un collimateur, est destinée à émettre, vers l'objet ou région d'objet à examiner 1, un rayonnement de spectre étroit, à fréquence centrale déterminée mais choisie à volonté. Ce rayonnement est émis sous la forme d'un faisceau cylindrique 10 d'axe moyen 11 et contenant un nombre connu $n_0$ de photons, et la source 2 a une énergie suffisante pour que la diffusion Compton (déviation d'un photon d'énergie lorsqu'il rencontre un électron dans la région traversée) soit l'effet prédominant de l'interaction entre ces photons émis et les électrons rencontrés par ceux-ci à l'intérieur de la région 1.

Le détecteur principal stationnaire 3, équipé de collimateurs 12 dont les axes longitudinaux respectifs sont perpendiculaires à l'axe moyen 11 du faisceau 10 émis par la source 2, est destiné à délivrer trois informations différentes : d'une part deux informations permettant un repérage de la position de chaque photons détecté, d'autre part une troisième information sous la forme d'un signal d'amplitude proportionnelle à l'énergie du photon détecté.

Le détecteur auxiliaire 4 est mis en place de façon que son axe longitudinal coïncide avec l'axe moyen 11 du faisceau et que la source 2 et ce détecteur, ainsi alignés, soient en outre situés de part et d'autre de la région à examiner 1.

Le procédé mis en œuvre dans l'appareil selon l'invention comprend alors les opérations suivantes :

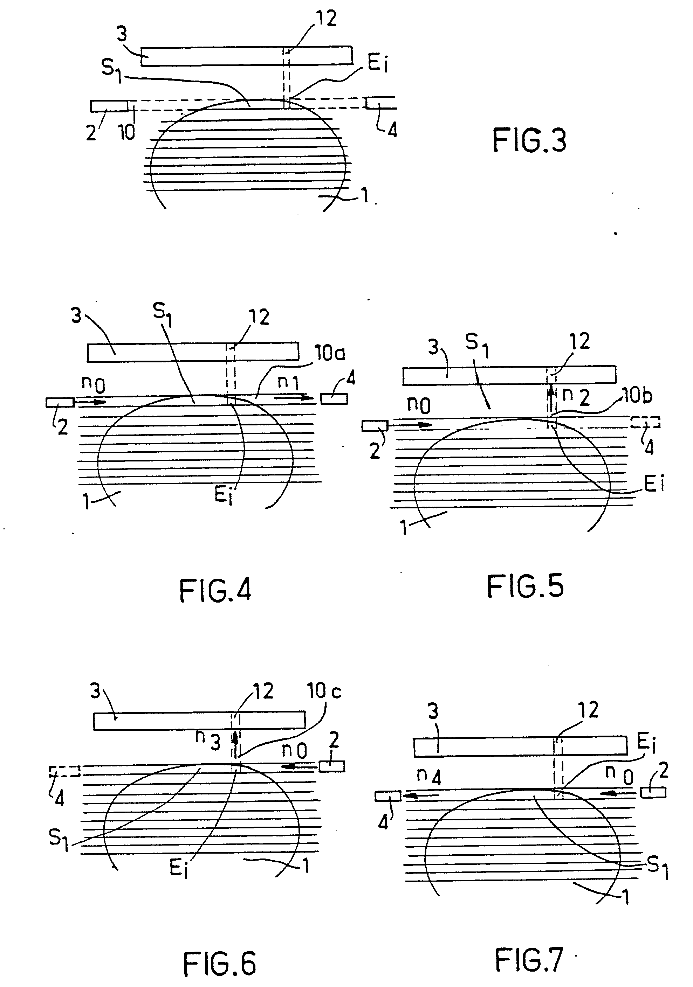

A) à l'aide de la source 2 et des détecteurs 3 et 4, on effectue trois mesures distinctes pour chaque élément de volume $E_i$ rencontré par le faisceau 10 dans la région à examiner, dans la tranche irradiée S1 la plus proche du détecteur principal stationnaire. Cette tranche S1 apparaît notamment sur la figure 3 et est perpendiculaire aux axes de collimateurs 12 du détecteur principal 3. L'irradiation est effectuée indifféremment par balayage linéaire (figure 1) ou circulaire (figure 2) autour de la région à examiner. Le repérage de position que permet ce détecteur 3 est défini par rapport à un repère de référence X, Y, Z visible sur les figures 1 et 2 et dont le plan X, Y est parallèle au plan moyen P de la tranche S1 (l'axe Z étant parallèle aux axes des collimateurs 12) et de toutes les tranches $S_j$ qui vont être successivement irradiées par la source 2. Les éléments de volume constituant chaque tranche sont des parallélépipèdes de petites dimensions définies par l'angle solide élémentaire de détection des collimateurs du détecteur principal et par l'épaisseur des tranches. Cette épaisseur a une valeur généralement voisine de celle du diamètre des collimateurs, mais peut être modifiée selon la nature de l'examen. Il est par exemple possible dans le cas d'examen de tissus biologiques d'effectuer un premier examen avec une épaisseur déterminée des tranches, pour connaître les coefficients d'absorption des tissus dans toute la région à examiner, puis de procéder à un deuxième examen plus détaillée d'une partie seulement de cette région, effectuée en adoptant une épaisseur des tranches réduite pour rendre l'analyse plus ·fine.

Les trois mesures distinctes associées à chaque élément de volume de la région à examiner sont les suivantes : (1) mesure du nombre $n_1$ des photons transmis de la source 2 au détecteur auxiliaire 4 en traversant tous les éléments de volume rencontrés (voir la figure 4, sur laquelle le faisceau de photons transmis qui atteint le détecteur 4 porte la référence 10a) dont, bien entendu, l'élément de volume $E_i$ plus particulièrement considéré, (2) mesure du nombre $n_2$ des photons diffusés par effet Compton de l'élément de volume $E_i$ vers le détecteur principal 3 (voir la figure 5, sur laquelle le faisceau de photons diffusés sensiblement à 90° qui atteint le détecteur 3 porte la référence 10b) et, (3) après permutation des positions respectives de la source 2 et du détecteur auxiliaire 4 par rapport à la région 1, mesure du nombre $n_3$ des photons diffusés par effet Compton du même élément de volume $E_i$ vers le détecteur principal 3 (voir la figure 6, sur laquelle le faisceau de photons diffusés sensiblement à 90° qui atteint le détecteur 3 porte la référence 10c). On notera bien, dès à présent, que, contrairement à celles de $n_2$ et de $n_3$, la mesure de $n_1$ est identique, à des fluctuations statistiques près, pour chacun des éléments de volume $E_i$ rencontrés par le faisceau 10 pour une position déterminée de la source 2 et du détecteur auxiliaire 4 associé. A titre de simplification, on n'effectue donc, pour cette position déterminée, qu'une seule mesure de $n_1$, valable pour chacun des éléments $E_i$ rencontrés. Cette remarque s'appliquera ici tout au long de la description, bien qu'il soit possible, évidemment, de maintenir une mesure distincte de $n_1$ pour chaque élément de volume rencontré.

B) à l'aide du dispositif de traitement des résultats de détection, dont la description détaillée apparaîtra plus loin, on prélève les résultats de ces trois mesures dès qu'ils sont fournis par les détecteurs principal 3 et auxiliaire 4, puis l'on calcule et stocke en mémoire la valeur exacte de la densité d'électrons dans chaque élément de volume $E_i$ de la tranche S1 examinée. Pour chaque élément $E_i$ d'une tranche quelconque, la densité $d_{i,j}(x, y, z)$ est donnée par la relation (1) de la forme :

$$d_{i,j} = \frac{n_2 + n_3}{K \cdot n_0 \cdot dx \cdot T_{i,j} \cdot \left( \sqrt{\dfrac{n_2}{n_3}} + \sqrt{\dfrac{n_3}{n_2}} \right) \cdot \sqrt{\dfrac{2\,n_1}{n_0}}} \tag{1}$$

Dans cette relation, $n_0$, $n_1$, $n_2$, $n_3$ ont déjà été définis, et dx représente la dimension de l'élément de volume dans le sens de propagation du faisceau incident 10 émis par la source 2. Le terme $T_{i,j}(x, y, z)$ fait, lui, intervenir la transmission du rayonnement diffusé à 90° par l'élément de volume considéré vers les tissus superposés présents entre cet élément de volume et le détecteur principal 3, et s'exprime par une relation (2) de la forme :

$$T_{i,j} = T_{i,j-1} \cdot e^{S \cdot d_{i,j-1} \cdot dz} \tag{2}$$

dans laquelle S représente la section efficace globale de diffusion, de l'épaisseur de chaque tranche $S_j$ de région à examiner, et $d_{i,j-1}(x, y, z)$ la valeur de densité d'électrons déjà calculée pour l'élément de volume de la tranche précédemment irradiée en contact avec l'élément de volume considéré. Selon l'invention, on procède à l'analyse de la région à examiner 1 en irradiant d'abord la tranche $S_1$ la plus proche du détecteur principal 3 : pour cette tranche, tous les photons diffusés à 90° par l'élément de volume considéré atteignent ce détecteur 3, ce qui entraîne que $T_{i,1}(x, y, z) = 1$. On peut alors effectuer le calcul rigoureux des valeurs de densité d'électrons dans chaque élément de volume de la tranche $S_1$, à partir de la relation (1).

4

C) dans chaque nouvelle tranche $S_j$ successivement rencontrée dans la région à examiner 1 en s'éloignant progressivement du détecteur principal 3, on répète les trois mesures distinctes de $n_1$, $n_2$ et $n_3$ effectuées en (A) et schématiquement représentées sur les figures 4, 5 et 6. A partir des nouveaux résultats de mesure obtenus et utilisés par le dispositif de traitement de données dès qu'ils lui sont fournis par les détecteurs principal 3 et auxiliaire 4, et également à partir des valeurs de densité d'électrons dans chaque élément de volume de la tranche $S_{j-1}$ précédemment irradiée, on calcule et stocke en mémoire la valeur exacte de la densité d'électrons dans chaque élément de volume de cette nouvelle tranche $S_j$. Cette densité est donnée par la relation (1) déjà mentionnée dans laquelle le terme $T_{i,j}(x, y, z)$ est donné par la relation (2) également citée. Le processus est identique pour chaque tranche, et il est manifeste maintenant que les densités peuvent être calculées pour chaque élément de volume d'une tranche si elles l'ont déjà été pour chaque élément de volume des tranches précédentes (c'est-à-dire situées entre elle et le détecteur principal 3).

D) à l'aide du dispositif de représentation, les valeurs stockées de densité d'électrons correspondant à une tranche quelconque $S_j$ de la région examinée 1 peuvent être affichées. On a de la sorte obtenu la représentation d'une image, ou carte, de la répartition des densités d'électrons dans une tranche quelconque de l'objet ou région d'objet examinée, et ce différemment de ce qui est prévu dans le brevet français cité plus haut puisque, dans le premier cas, on veut recueillir à l'aide d'un diaphragme de forme appropriée le maximum de photons diffusés suivant un angle quelconque déterminé par rapport au faisceau d'émission, alors que, dans l'autre, on recherche au contraire une sélection sévère du nombre de ces photons, y compris l'élimination quasi-totale des photons diffusés plusieurs fois.

Au cours de la mise en œuvre de l'appareil conforme à l'invention, une quatrième mesure peut être éventuellement effectuée en association avec les trois mesures de $n_1$, $n_2$, $n_3$ déjà citées. Cette mesure supplémentaire est celle du nombre $n_4$ de photons qui, lorsqu'on a effectué la permutation des positions respectives de la source 2 et du détecteur auxiliaire 4 par rapport à la région 1, sont transmis de cette source à ce détecteur en traversant tous les éléments de volume rencontrés (dont, bien entendu, le même élément de volume $E_i$ déjà considéré pour les trois premières mesures). La valeur $n_4$ ainsi obtenue devrait théoriquement être identique à celle de $n_1$. Les différences constatées dans la réalité sont dues aux légères différences de collimation qui peuvent apparaître d'une mesure à l'autre, à l'influence résiduelle des photons plusieurs fois diffusés, et surtout aux fluctuations statistiques liées à la nature même de la mesure. Cette quatrième mesure, à laquelle s'adresse également la remarque effectuée à la fin du paragraphe (A) ci-dessus relativement au nombre de mesures nécessaire, sert donc à disposer d'une valeur moyenne $(n_1 + n_4)/2$ qui, dans la relation (1), vient remplacer $n_1$.

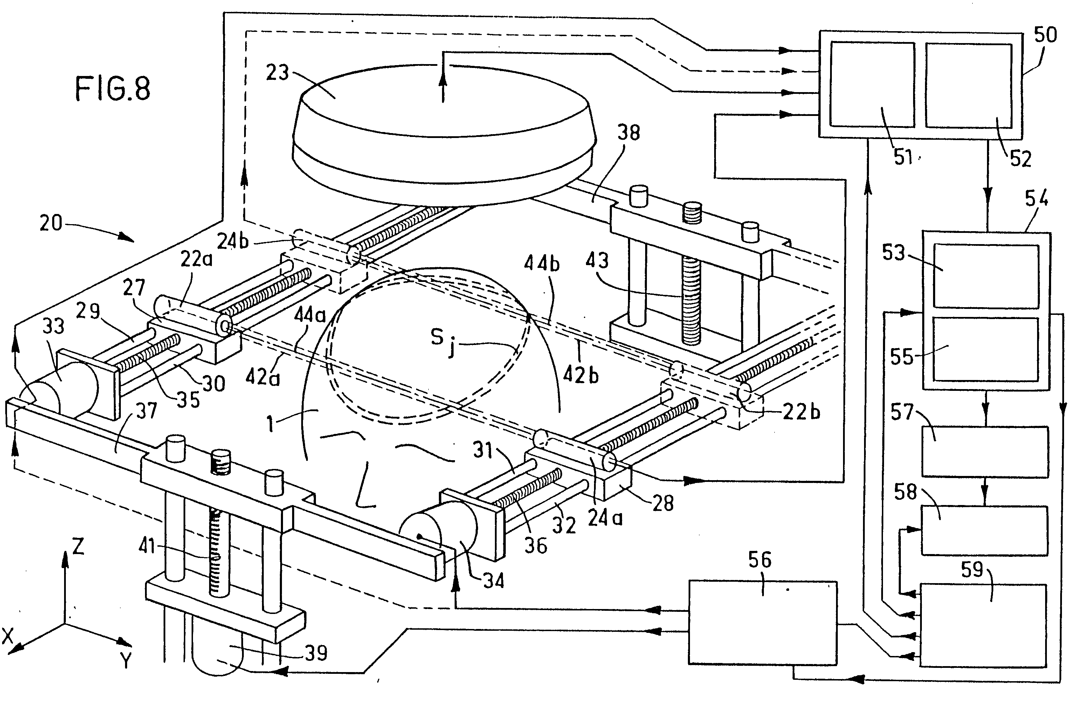

Un appareil d'examen tomographique 20 pour la mise en œuvre du procédé exposé ci-dessus est maintenant décrit de façon plus détaillée en se référant à la figure 8. Cet appareil 20 comprend une source de rayons gamma 22a (par exemple une source de cobalt 57), un détecteur principal de rayonnement 23, qui est une caméra de gammagraphie, et un détecteur auxiliaire de rayonnement 24a, qui est un scintillateur individuel couplé à un tube photomultiplicateur individuel. La source 22a est équipée d'un collimateur, et le détecteur principal 23 de collimateurs parallèles entre eux et perpendiculaires à l'axe 44a du faisceau 42a émis par cette source. On supposera dans toute la description que la surface de ce détecteur principal 23 est suffisante pour que tout l'objet à examiner, dans le cas présent la région 1, soit interceptée par les angles solides élémentaires de détection des collimateurs du détecteur principal 23.

La source 22a et le détecteur auxiliaire 24a sont alignés et portés par des supports 27 et 28, eux-mêmes soutenus par des tiges de guidage 29, 30, 31 et 32 respectivement. Ces supports 27 et 28 peuvent être simultanément déplacés pour irradier une tranche déterminée de région à examiner suivant une première direction de balayage perpendiculaire aux axes de détection du détecteur principal 23 (les axes longitudinaux des collimateurs) ; ce déplacement s'effectue sous l'action de moteurs 33 et 34 entraînant en rotation des tiges filetées 35 et 36 respectivement. Les tiges 35 et 36 traversent les supports 27 et 28 respectivement et les entraînent de façon synchrone afin de conserver l'alignement de la source 22a et du détecteur auxiliaire 24a.

Des bras transversaux 37 et 38 constituent l'armature de l'appareil, qui peut à son tour être déplacé suivant une deuxième direction de balayage parallèle aux axes de détection du détecteur principal 23, sous l'action des moteurs 39 et 40 (le moteur 40 n'est pas visible sur la figure 8) entraînant des tiges filetées 41 et 43 respectivement. Ces tiges 41 et 43 assurent l'élévation ou l'abaissement des bras transversaux 37 et 38 respectivement, cet entraînement étant lui aussi effectué de façon synchrone afin de maintenir parallèles les tranches successivement irradiées au cours de ce balayage.

Pour chaque élément de volume $E_i$ considéré dans une tranche de région à examiner, par exemple la tranche $S_j$ représentée sur la figure 8, les mesures des nombres $n_1$, $n_2$, $n_3$ définis précédemment sont effectuées de la façon suivante. Le détecteur auxiliaire 24a aligné avec la source 22a fournit le nombre $n_1$ des photons du faisceau cylindrique 42a délivré par la source 22a qui atteignent ce détecteur. Le détecteur principal stationnaire 23 permet d'obtenir le nombre $n_2$ des photons diffusés vers lui par effet Compton. Par ailleurs, un deuxième ensemble d'émission-détection, constitué d'une deuxième source 22b (émettant un faisceau de photons 42b d'axe moyen 44b) et d'un deuxième détecteur auxiliaire 24b (également un scintillateur individuel) et placé parallèlement au premier ensemble 22a, 24a mais tête-

5

bêche par rapport à lui, est prévu pour permettre la mesure de $n_3$ et, éventuellement, celle de $n_4$ : le nombre $n_3$ est fourni par le détecteur principal 23, le nombre $n_4$ est fourni par le deuxième détecteur auxiliaire 24b lorsque celui-ci occupe la position qui était celle de la source 22a au moment de la mesure de $n_1$. On a vu plus haut que l'utilisation de $n_4$ est en quelque sorte facultative, son avantage étant uniquement de permettre le remplacement, dans la relation (1) d'une valeur unique $n_1$ par une valeur moyenne $(n_1 + n_4)/2$.

Sur la figure 8, la source 22b et le détecteur auxiliaire 24b ont été représentés en pointillé. En effet, au lieu de prévoir ce deuxième ensemble d'émission-détection, on pourrait utiliser le même premier ensemble 22a, 24a pour effectuer les mesures de $n_3$. Dans ce cas, après avoir effectué un premier balayage d'une tranche, au cours duquel sont mesurés $n_1$ et $n_2$, on fait pivoter l'appareil de 180° autour de la région à examiner de façon à permuter les positions de la source 22a et du détecteur auxiliaire 24a. Dans cette nouvelle position, on peut effectuer, toujours à l'aide des moteurs 33 et 34, un deuxième balayage de la même tranche, permettant la mesure de $n_3$ et, éventuellement, de $n_4$.

Les résultats des mesures de $n_1$, $n_2$, $n_3$ et éventuellement de $n_4$, sont prélevés par une unité d'acquisition de données 50 ; celle-ci comprend une unité d'échange d'informations 51 destinée à écrire dans une mémoire auxiliaire 52 les nombres $n_1$ (fourni par le détecteur auxiliaire 24a), $n_2$ (fourni par le détecteur principal 23), $n_3$ (fourni également par le détecteur 23), $n_4$ (éventuellement fourni soit par le détecteur auxiliaire 24b s'il est prévu, soit, dans le cas contraire, par le détecteur auxiliaire 24a), une coordonnée y (fournie par le détecteur principal 23), et une coordonnée x (fournie par la position des moteurs 33 et 34). Ces coordonnées sont définies dans un repère (OX, OY, OZ) d'axes deux à deux perpendiculaires, représenté en bas et à gauche de la figure 8. Ces grandeurs $n_1$, $n_2$, etc... sont inscrites en mémoire auxiliaire 52 au fur et à mesure de leur acquisition au cours de l'irradiation de la tranche examinée, ici $S_j$.

Lorsque l'acquisition de données est terminée pour la tranche $S_j$, ces données sont envoyées par l'unité d'échange d'informations 51 vers la mémoire centrale 53 d'un calculateur 54 et utilisées par une unité de calcul 55 pour déterminer la valeur de densité électronique dans chacun des éléments de volume $E_i$ de la tranche $S_j$ irradiée. Pendant ce temps, et dès que la mémoire centrale 53 est terminée, le calculateur 54 envoie vers le dispositif de commande de balayage 56 l'ordre de passer à l'examen (et donc à l'irradiation) de la tranche suivante $S_{j+1}$, par commande du fonctionnement des moteurs 39 et 40. De la sorte, l'analyse de cette tranche $S_{j+1}$ et l'introduction en mémoire auxiliaire 52 des résultats de mesure correspondants (x, y, $n_1$, $n_2$, $n_3$, et éventuellement $n_4$) sont réalisées pendant le calcul des valeurs de densité concernant la tranche $S_j$.

Ce calcul, on l'a vu, utilise des valeurs précédemment calculées, à savoir $T_{j-1}$ et $d_{i,j-1}$, qui doivent donc être maintenues dans la mémoire centrale 53 jusqu'à ce qu'elles aient été utilisées. Lorsque les calculs concernant la tranche $S_j$ sont terminés, les nouvelles valeurs $T_j$ et $d_{i,j}$ viennent remplacer dans la mémoire centrale 53 les valeurs $T_{j-1}$ et $d_{i,j-1}$ qui s'y trouvaient (ces nouvelles valeurs vont en effet être utilisées dans tous les calculs concernant la tranche $S_{j+1}$). Pendant ce temps, les valeurs calculées $d_{i,j}$ de densité d'électrons sont transférées d'une part sur disque magnétique dans une mémoire de stockage 57 et d'autre part vers la mémoire d'image d'une console de visualisation 58.

Lorsque tous les résultats sont acquis et toutes les valeurs de densité calculées pour toute la région à examiner, le stockage de ces valeurs sur disque magnétique offre à l'utilisateur (par exemple, au médecin en quête de diagnostic) la possibilité d'effectuer à tout instant le transfert des valeurs de densité d'une tranche quelconque vers la mémoire d'image de la console de visualisation 58, dans le but de permettre une comparaison entre différentes tranches de la région examinée.

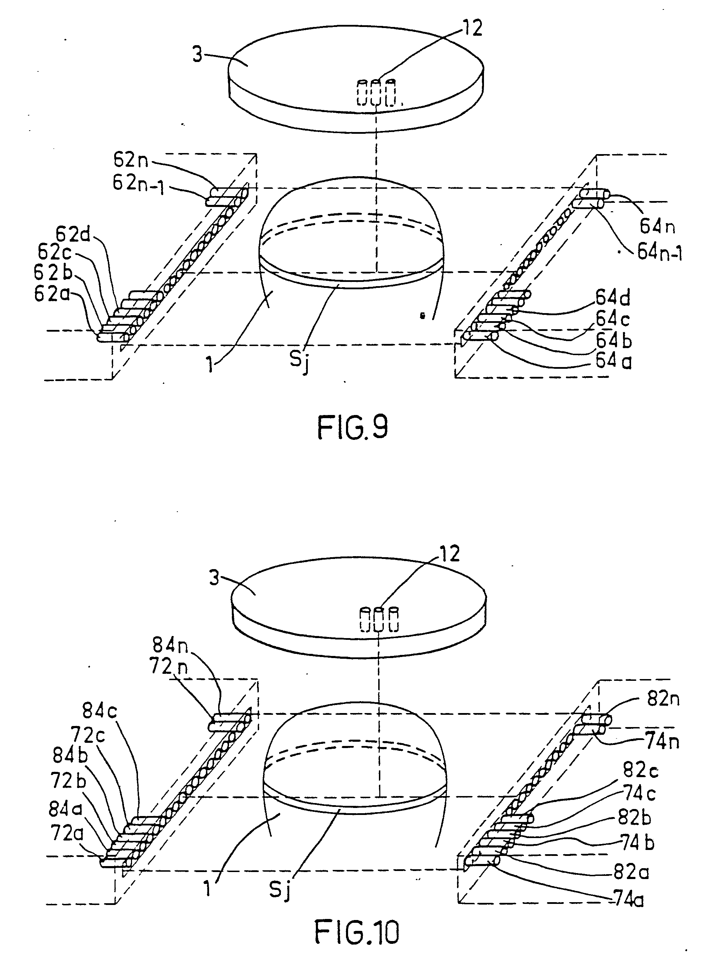

Trois autres modes de réalisation de l'appareil selon l'invention peuvent être plus particulièrement envisagés et sont décrits ci-dessous en se référant aux figures 9 à 11.

Le deuxième mode de réalisation proposé (figure 9) est analogue à celui représenté sur la figure 8, à l'exception suivante près : au lieu d'avoir prévu un seul ensemble d'émission-détection (22a, 24a), ce sont n ensembles identiques juxtaposés (62a, 64a), (62b, 64b),...., (62i, 64i),...., (62n, 64n) qui assurent d'une part l'irradiation de chaque tranche à examiner et d'autre part la mesure du nombre $n_1$ des photons de chaque faisceau transmis à travers la région à examiner. Cette variante de réalisation présente l'avantage de remplacer le dispositif de balayage mécanique suivant la première direction par un ensemble stationnaire de sources et de détecteurs auxiliaires, associé à un dispositif de commutation des sources 62a à 62n (un tel dispositif de commutation est de type connu et n'est ici ni décrit ni représenté).

Le troisième mode de réalisation proposé (figure 10) ne diffère également de ceux des figures 8 et 9 que par la caractéristique suivante : on prévoit d'une part n premiers ensembles d'émission-détection identiques (72a, 74a) à (72n, 74n), qui assurent, pour chaque tranche à examiner, l'irradiation au cours de laquelle sont mesurés les nombres $n_1$ et $n_2$, et d'autre part n deuxièmes ensembles d'émission-détection (82a, 84a) à (82n, 84n), identiques entre eux et aux n premiers, mais placés tête-bêche par rapport à ceux-ci et de façon à imbriquer de façon régulièrement alternée les faisceaux émis par les sources 72a à 72n et 82a à 82n. Ces n deuxièmes ensembles assurent, pour chaque tranche à examiner, l'irradiation au cours de laquelle sont mesurés les nombres $n_3$ et peuvent être éventuellement mesurés les nombres $n_4$. Dans cette variante de réalisation, la complexité sensiblement accrue de l'appareil est avantageusement compensée par la suppression, au cours des mesures, de l'opération de permutation source-détecteur (qui était nécessaire pour effectuer les mesures de $n_3$ et $n_4$ après avoir effectué celles de $n_1$ et $n_2$), et la

quasi-suppression du balayage suivant la première direction. Ce balayage peut en effet être remplacé par un simple décalage, dans la même direction, de la largueur du faisceau émis par les sources ; obtenu soit par décalage de tous les ensembles d'émission-détection parallèlement au plan de la tranche examinée soit par déplacement du support de l'objet à examiner et du détecteur principal de la même valeur.

Le décalage suivant la première direction de balayage peut même être totalement supprimé si les n premiers et les n deuxièmes ensembles d'émission-détection (72a à n, 74a à n) et (82a à n, 84a à n) sont placés non plus dans le plan d'une même tranche, mais de façon à irradier les uns une tranche $S_{j-1}$ et les autres la tranche suivante $S_j$. Les sources de l'une ou l'autre de ces deux séries de n ensembles peuvent alors à nouveau être juxtaposées, et les faisceaux qu'elles émettent être jointifs. Dans le cas de cette solution, l'acquisition des informations concernant une tranche $S_j$ n'est bien entendu terminée que lorsque cette tranche a été irradiée successivement par les n premiers puis les n deuxièmes ensembles d'émission-réception ; cette solution implique aussi une synchronisation précise du dispositif de commutation des sources et de la désignation des emplacements de mémoire correspondant à chaque élément de volume.

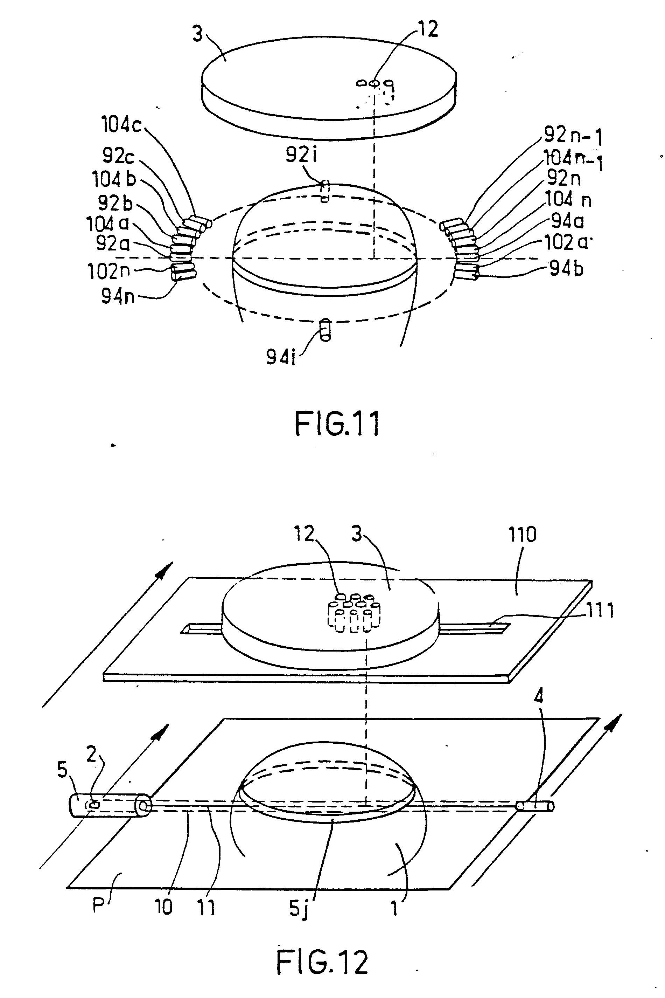

Un quatrième mode de réalisation de l'appareil selon l'invention est proposé sur la figure 11. Sa structure et son fonctionnement sont analogues à ceux de l'appareil représenté sur la figure 10, à cette différence près que les n premiers et deuxièmes ensembles d'émission-détection, respectivement (92a à 92n, 94a à 94n) et (102a à 102n, 104a à 104n), sont répartis autour de la région à examiner 1 selon une disposition circulaire. Comme dans le cas du troisième mode de réalisation, cette variante est caractérisée par le remplacement, au cours des mesures, de l'opération de permutation source-détecteur par une simple opération de décalage circulaire de la largeur d'un faisceau. Ce décalage peut d'ailleurs, comme précédemment, être supprimé si les n premiers ensembles sont placés de façon à irradier une tranche $S_{j-1}$ et les n deuxièmes ensembles de façon à irradier une tranche $S_j$.

Dans la description qui précède, on a supposé jusqu'à présent que seuls la ou les sources de rayons gamma et le détecteur principal étaient équipés de collimateurs. Il est cependant avantageux de prévoir également un collimateur pour chacun des détecteurs auxiliaires, car chacun de ceux-ci est susceptible de recevoir non seulement les photons qui lui sont transmis par la source correspondante alignée avec lui, mais aussi ceux qui, bien que porteurs d'une énergie plus faible, lui parviennent après avoir subi à l'intérieur de la région examinée 1 plusieurs diffusions successives par effet Compton. Ce flux de photons diffusés perturbe les mesures de $n_1$ et $n_4$. La présence d'un collimateur devant chaque détecteur auxiliaire réduit très notablement l'influence de ces photons au moins deux fois diffusés : en effet il rend le détecteur auxiliaire auquel il est associé insensible à tout rayonnement qui ne l'atteint pas suivant une direction parallèle à son axe longitudinal.

La présence de ce collimateur apporte en outre la possibilité de modifier l'ouverture du faisceau émis par la ou les sources, par suppression ou modification du collimateur de celles-ci ; cette modification ne sera cependant envisagée que dans un plan parallèle à chaque tranche exclusivement, la limitation d'ouverture de ce même faisceau d'émission dans la direction perpendiculaire aux plans des tranches restant indispensable pour définir l'épaisseur de ces tranches. Cette possibilité de modification est notamment utile dans le cas où l'on veut augmenter la vitesse d'examen en irradiant simultanément une plus grande surface de la région à examiner et en sollicitant non plus un mais plusieurs détecteurs auxiliaires en même temps.

L'influence des photons plusieurs fois diffusés est également sensible sur les mesures effectuées par le détecteur principal stationnaire. Pour l'atténuer notablement, on peut équiper ce détecteur d'un dispositif de sélection énergétique. Comme les photons perdent une fraction de leur énergie lors de chacune des diffusions par effet Compton qui résultent des interactions photons-électons dans la région à examiner, l'énergie de la plupart des photons plusieurs fois diffusés qui atteignent le détecteur principal est différente de celles de photons diffusés une seule fois. En adjoignant à la caméra de gammagraphie un circuit électonique qui joue le rôle de fenêtre énergétique, on peut réaliser une sélection énergétique qui évite de prendre en compte, au cours de mesures des nombres $n_2$ et $n_3$, ces photons plusieurs fois diffusés.

On peut également diminuer l'influence des photons plusieurs fois diffusés sur les mesures de $n_2$ et $n_3$ en utilisant comme détecteur principal stationnaire une chambre à fils comprenant des électrodes de directions parallèles aux axes OX et OY respectivement. Les électrodes parallèles à OY sont en état de blocage, et une impulsion électrique est envoyée successivement sur chacune d'entre elles, dans le but de débloquer électriquement la zone correspondante du détecteur située exactement en regard du faisceau émis par la source en activité à cet instant, c'est-à-dire située dans le plan défini par le faisceau et les axes de détection du détecteur principal qui interceptent ce faisceau. Cette zone dans laquelle est réalisé le déblocage électrique du détecteur principal se déplace en synchronisme avec le mouvement de balayage suivant la première direction de balayage ; on ne détecte plus, maintenant, que les photons plusieurs fois diffusés dont la trajectoire est contenue dans ce plan défini par le faisceau et les axes de détection. Tous les autres photons diffusés au moins deux fois ne sont plus pris en compte.

Un résultat sensiblement identique est obtenu si l'on utilise un détecteur principal sans blocage local comme celui qui vient d'être décrit, mais en revanche équipé d'un obturateur mécanique tel que l'obturateur 110 visible sur la figure 12. Un tel obturateur, réalisé par exemple en plomb, ou en tout autre matériau de numéro atomique élevé (tungstène, tantale), est percé d'une fente rectiligne 111 et se

7

déplace devant le détecteur principal en synchronisme avec l'ensemble d'émission-détection en activité à cet instant. Cet obturateur 110 absorbe tous les rayons gamma qui ne se présentent pas en face de la fente rectiligne 111.

Bien entendu, la présente invention n'est pas limitée aux exemples de réalisation qui viennent d'être décrits et représentés, à partir desquels on pourra prévoir d'autres modes et d'autres formes de réalisation sans pour cela sortir du cadre de l'invention. En particulier, les sources de cobalt 57 ont été choisie pour équiper l'appareil parce qu'elles sont caractérisées par un flux élevé de photons par unité de volume actif et par la présence d'un seul pic d'émission, à 122 keV. Ce choix n'est cependant pas limitatif et d'autres sources de rayons X ou gamma peuvent être utilisées.

Par ailleurs, la caméra de gammagraphie choisie comme détecteur principal peut être plus spécialement de type Anger, ce qui permet d'utiliser, si nécessaire, le dispositif de sélection énergétique dont ce type de caméra est équipé.

**Revendications**

1. Appareil d'examen tomographique aux rayons X ou gamma pour le contrôle de la structure interne d'un objet tridimensionnel (1), par représentation d'images de tranches de cet objet à l'aide d'une carte de la densité d'électrons dans des éléments de volume de ces tranches, cet appareil comprenant :

(a) au moins une source (22a) de rayons X ou gamma équipée d'un collimateur pour émettre vers ledit objet un faisceau sensiblement cylindrique (42a) porteur d'une énergie telle que la diffusion Compton soit l'effet prédominant de l'interaction entre les photons ainsi émis et les électrons rencontrés par ceux-ci dans l'objet ;

(b) au moins un détecteur auxiliaire (24a) de rayonnement X ou gamma transmis, situé par rapport à la source de l'autre côté de l'objet et aligné avec elle pour constituer avec celle-ci un ensemble d'émission-détection déplaçable par rapport à l'objet à examiner ;

(c) un détecteur principal (23) de rayonnement X ou gamma équipé de collimateurs ;

(d) un dispositif de balayage de l'objet à examiner par ledit faisceau émis ;

(e) un dispositif (50, 54, 57) de traitement des résultats de détection ;

(f) un dispositif (58) de visualisation des valeurs calculées et stockées dans ledit dispositif de traitement ;

ledit appareil étant caractérisé :

(A) en ce que le détecteur principal (23) est stationnaire et en ce que, pour permettre le repérage de la position de chaque photon détecté, ses collimateurs sont parallèles en vue de définir des axes de détection parallèles entre eux et perpendiculaires à l'axe moyen du faisceau (42a), ces collimateurs étant prévus en nombre suffisant pour que le champ de vue de ce détecteur couvre toute la région d'objet à examiner ;

(B) en ce qu'il comprend un dispositif (56) de commande de balayage d'une part suivant une première direction dans un plan parallèle à celui des tranches d'objet successivement examinées et d'autre part suivant une deuxième direction de balayage transversale ou perpendiculaire à ce plan, ladite première direction de balayage étant prévue pour permettre, pour les $n \times m$ éléments de volume de la tranche d'objet à examiner rencontrés par le faisceau (42a) au cours du balayage des $m$ positions successives de ce faisceau, l'obtention de $m$ mesures du nombre de photons transmis de la source (22a) au détecteur auxiliaire (24a) et de $n \times m$ mesures du nombre de photons diffusés par effet Compton de chaque élément de volume rencontré par ledit faisceau vers le détecteur principal stationnaire (23) et, après permutation des positions respectives de ladite source et dudit détecteur auxiliaire par des moyens mécaniques ou électroniques, l'obtention de $n \times m$ nouvelles mesures du nombre de photons diffusés par effet Compton de chaque élément de volume vers ledit détecteur principal stationnaire, et ladite deuxième direction de balayage étant prévue pour permettre la reproduction de $m (2n + 1)$ nouvelles mesures similaires pour chaque élément de volume de chaque nouvelle tranche $S_j$ successivement irradiée dans l'objet à examiner (1) en s'éloignant progressivement dudit détecteur principal stationnaire ;

(C) en ce que le dispositif de traitement des résultats de détection (50, 54, 57) est prévu pour permettre : (i) à partir des $m (2n + 1)$ mesures dans la première tranche irradiée, la détermination de la valeur de la densité des électrons dans chaque élément de volume de cette tranche, et (ii) à partir d'une part desdites $m (2n + 1)$ mesures dans chaque nouvelle tranche $S_j$ successivement rencontrée dans la deuxième direction de balayage et d'autre part des valeurs de densité d'électrons calculées par ledit dispositif de traitement pour chaque élément de volume de tranche précédemment irradiée $S_{j-1}$, la détermination de la valeur de la densité des électrons dans chaque élément de volume de ces nouvelles tranches, l'ensemble desdites valeurs déterminées étant alors stocké en mémoire ; et

(D) en ce que le dispositif (58) de visualisation est destiné à permettre la représentation des valeurs de densité d'électrons ainsi calculées et stockées, sous la forme d'une carte de densité associée à l'une quelconque des tranches d'objet examiné.

2. Appareil selon la revendication 1, caractérisé en ce que ladite première direction de balayage est circulaire.

3. Appareil selon l'une des revendications 1 et 2, caractérisé en ce qu'il comprend une série de sources (62i) de rayons X ou gamma et une série de détecteurs (64i) de rayonnement transmis respectivement alignés avec ces sources pour constituer avec elles une série d'ensembles d'émission-détection identiques répartis suivant la première direction de balayage, un dispositif de commutation des sources suivant ladite première direction étant associé à ces ensembles.

4. Appareil selon l'une des revendications 1 à 3, caractérisé en ce qu'on associe au détecteur principal stationnaire (23) un obturateur percé d'une fente rectiligne parallèle aux faisceaux émis, cet obturateur étant mobile suivant la première direction de balayage.

**Claims**

1. An apparatus for tomographic examination of the internal structure of a three-dimensional object (1) by means of X-rays or gamma rays for the display of images of slices of the object with the aid of a representation of the electron density in volume elements of said slices, which apparatus includes :

(a) at least one X-ray or gamma ray source (22a) which includes a collimator and which serves to emit to said object a substantially cylindrical beam (42a) which carries an energy such that Compton diffusion is the predominant effect in the interaction between the photons thus emitted and the electrons encountered thereby in the object ;

(b) at least one auxiliary detector (24a) for emitted X-rays or gamma rays which is situated at the other side of the object with respect to the source and which is aligned with respect thereto in order to form in conjunction therewith an emission/detection assembly which is displaceable with respect to the object to be examined ;

(c) a principal detector (23) for X-rays or gamma rays which is provided with collimators ;

(d) a device for scanning the object to be examined by means of said emitted beam ;

(e) a device (50, 54, 57) for processing the detection results ;

(f) a device (58) for displaying the values calculated and stored in said processing device ; which apparatus is characterized in that :

(A) the principal detector (23) is stationary and in that, in order to enable the localization of each photon detected, its collimator extend in parallel so as to define parallel detection axes therebetween which are perpendicular to the central axis of the beam (42a) there being provided such a number of collimators that the field of vision of the detector covers any region of the object to be examined ;

(B) it includes a device (56) for controlling the scanning on the one hand in a first direction in a plane parallel to that of the slices of the object which are successively examined, and on the other hand in a second scanning direction which is transverse or perpendicular to this plane, said first scanning direction serving to enable the execution, for the $n \times m$ volume elements of the object slice to be examined which are encountered by the beam (42a) during the scanning of the m successive positions of this beam, of m measurements of the number of photons by the source (22a) to the auxiliary detector (24a) and of $n \times m$ measurements of the number of photons diffused by the Compton effect by each volume element encountered by said beam in the direction of the stationary principal detector (23) and, after permutation of the respective positions of said source and said auxiliary detector by mechanical or electronic means, the execution of $n \times m$ new measurements of the number of photons diffused by the Compton effect by each volume element towards said stationary principal detector, said second scanning direction serving to enable the reproduction of m $(2n + 1)$ new, similar measurements for each volume element of each new slice Sj successively irradiated in the object (1) to be examined, said slice being situated successively further from said stationary principal detector ;

(C) the device (50, 54, 57) for processing the detection results being provided to enable : (i) determination, on the basis of the m $(2n + 1)$ values in the first slice irradiated , the value of the electron density in each volume element of the relevant slice, and (ii) determination on the basis of on the one hand said values m $(2n + 1)$ in each new slice Sj successively encountered in the second scanning direction and on the other hand the electron density values calculated by said processing device for each volume element of slice $(S_{j-1})$ previously irradiated, the value of the electron density in each volume element of these new slices, the set of values thus determined being stored in the memory ; and

(D) the display device (58) is suitable for the display of the electron density values thus calculated in the form of a representation of the density associated with any one of the object slices examined.

2. An apparatus as claimed in Claim 1, characterized in that said first scanning direction is circular.

3. An apparatus as claimed in one of the Claims 1 and 2, characterized in that it includes a series of X-ray or gamma ray sources (62i) and a series of detectors (64i) for the transmitted radiation which are aligned with said sources in order to form, is conjunction therewith, a series of identical emission/detection assemblies distributed in the first scanning direction, a device for switching over the sources in said first direction being associated with said assemblies.

4. An apparatus as claimed in any of the Claims 1 to 3, characterized in that with the stationary principal detector (23) there is associated and obturator which is provided with a rectilinear slit parallel to the beams emitted, said obturator being displaceable in the first scanning direction.

# 0 011 897

**Patentansprüche**

1. Gerät zur tomographischen Abtastung mittels Röntgen- oder Gamma-Strahlen für die Untersuchung der inneren Struktur eines dreidimensionalen Gegenstandes (1) für die bildliche Wiedergabe von Schnitten dieses Gegenstandes mit Hilfe einer Aufnahme der Elektronendichte in den volumenelementen dieser Schnitte, das enthält :

(a) zumindest eine Röntgen- oder Gamma-Strahlungsquelle (22a) mit einem Kollimator zum Aussenden eines in wesentlichen zylindrischen Strahls (42a) als Träger einer derartigen Energie zum genannten Objekt, dass der Compton-Effekt bei der Wechselwirkung zwischen den so ausgesandten Photonen und den damit zusammenstössenden Elektronen im Objekt dominiert ;

(b) zumindest einen Röntgen- oder Gamma-Strahlungshilfsdetektor (24a), der in bezug auf die Strahlungsquelle an der anderen Seite des Objekts angeordnet und darauf ausgerichtet ist und mit ihr eine gegen das zu untersuchende Objekt verschiebbare Emissions-Detektions-Kombination bildet ;

(c) einen Röntgen- oder Gamma-Hauptdetektor (23) mit Kollimatoren ;

(d) eine Abtasteinrichtung zum Abtasten des zu untersuchenden Objekts mit dem genannten Strahl ;

(e) eine Einrichtung (50, 54, 57) zum Verarbeiten der Detektorergebnisse,

(f) eine Einrichtung (58) zum Anzeigen der berechneten und in der genannten Verarbeitungseinrichtung gespeicherten Werte,

dadurch gekennzeichnet, dass

(A) dass der Hauptdetektor (23) stationär ist und dass zum Orten der Position jedes detektierten Photons seine Kollimatoren im Hinblick auf die Definition der untereinander parallelen und senkrecht zur mittleren Achse des Strahls (42a) verlaufenden Detektionsachsen parallel verlaufen, wobei diese Kollimatoren in einer genügenden Anzahl vorhanden sind, um das Arbeitsfeld dieses Detektors das ganze zu untersuchende Objektgebiet erfassen zu lassen,

(B) dass eine Einrichtung (56) zur Steuerung der Abtastung einerseits in einer ersten Richtung in einer zu den aufeinanderfolgend untersuchten Objektschnitten parallel verlaufenden Ebene und zum andern in einer zweiten transversal oder senkrecht zu dieser Ebene verlaufenden vorgesehen ist, wobei diese erste Abtastrichtung dazu vorgesehen ist, für die während der Abtastung der m aufeinanderfolgenden Positionen dieses Strahls vom Strahl (42a) getroffenen n × m Volumenelemente des Schnitts des zu untersuchenden Objekts m Messungen der von der Strahlungsquelle (22a) auf den Hilfsdetektor (24a) übertragenen Photonenanzahl sowie n × m Messungen der Zahl der durch den Compton-Effekt jedes Volumenelements vom genannten Strahl zum stationär Hauptdetektor (23) gestreuten Photonen und nach dem Tausch der jeweilige Positionen der Strahlungsquelle und des Hilfsdetektors mit mechanischen oder elektronischen Mitteln n × m neuen Messungen der durch den Compton-Effekt jedes Volumenelements zum stationären Hauptdetektor ausgestrahlten Photonenanzahl durchzuführen und wobei die zweite Abtastrichtung zum Reproduzieren von m (2n + 1) neuen gleichen Messungen für jedes Volumenelement jedes in dem zu untersuchenden Objekt (1) während der progressiven Entfernung des genannten stationären Hauptdetektors aufeinanderfolgend durchstrahlten neuen Schnittes Sj vorgesehen ist,

(C) dass die Verarbeitungseinrichtung der Detektorergebnisse (50, 54, 57) dazu vorgesehen ist, (i) ausgehend von den m (2n + 1) Messungen im ersten durchstrahlten Schnitt den Wert der Elektronendichte in jedem Volumenelement dieses Schnitts zu bestimmten, und (ii) ausgehend einerseits von den genannten m (2n + 1) Messungen in jedem in der zweiten Abtastrichtung nacheinander getroffenen neuen Schnitt (Sj) und andrerseits von den von der genannten Verarbeitungseinrichtung für jedes Volumenelement der zuvor durchstrahlten Schicht $S_{j-1}$ berechneten Elektronendichtewerten, den Wert der Elektronendichte in jedem Volumenelement dieser neuen Schnitte zu bestimmen, wobei die Gesamtheit dieser bestimmten Werte gespeichert wird, und

(D) dass die Anzeigeeinrichtung (58) dazu bestimmt ist, die Darstellung der auf diese Weise berechneten und gespeicherten Werte der Elektronendichte in Form einer Kartenaufnahme der jedem der Schnitte des untersuchten Objekts zugeordneten Dichte zu ermöglichen.

2. Gerät nach Anspruch 1, dadurch gekennzeichnet, dass die erste Abtastrichtung kreisförmig ist.

3. Gerät nach einem der Ansprüche 1 und 2, dadurch gekennzeichnet, dass es eine Reihe von Röntgen- oder Gamma-Strahlungsquellen (62i) und eine Reihe von auf diese Strahlungsquellen ausgerichteten Strahlungsdetektoren (64i) enthält, um damit eine Reihe von entsprechend der ersten Abtastrichtung verteilten identischen Emissions-Detektions-Kombinationen zu bilden, wobei eine Schaltvorrichtung der Strahlungsquellen entsprechend der genannten ersten Richtung diesen Kombination zugeordnet ist.

4. Gerät nach einem der Ansprüche 1 bis 3, dadurch gekennzeichnet, dass dem stationären Hauptdetektor (23) eine von einem parallel zu den Strahlen verlaufenden geradlinigen Schlitz durchlochte Blende geordnet ist, und diese Blende entsprechend der ersten Abtastrichtung bewegbar ist.

FIG.1

FIG.2

0 011 897

FIG.3

FIG.4

FIG.5

FIG.6

FIG.7

2

FIG.8

FIG.9

FIG.10

FIG.11

FIG.12

5